# EUROPÄISCHE PATENTSCHRIFT

(11) **EP 3 928 860 B1**
(45) Veröffentlichungstag und Bekanntmachung des Hinweises auf die Patenterteilung: **06.08.2025**
(21) Anmeldenummer: 20182068.5
(22) Anmeldetag: 24.06.2020
(51) Int. Cl.: B01J 13/14, A61K 9/50, C09B 67/02, C11D 3/50

(54) **VERFAHREN ZUR HERSTELLUNG VON MIKROPARTIKELN**
METHOD FOR PRODUCING MICROPARTICLES
PROCÉDÉ DE FABRICATION DE MICROPARTICULES

(43) Veröffentlichungstag der Anmeldung: 29.12.2021
(73) Patentinhaber: Universität des Saarlandes, 66123 Saarbrücken (DE)
(72) Erfinder: Kickelbick, Guido, 66386 St. Ingbert (DE); Steinbrück, Nils, 93173 Wenzenbach (DE); Pohl, Svenja, 66111 Saarbrücken (DE)
(74) Vertreter: Epping - Hermann - Fischer

(56) Entgegenhaltungen:
- EP-A2- 1 130 046
- US-A- 5 064 894
- US-A1- 2001 025 080
- US-A1- 2011 135 888
- DATABASE WPI Week 200650, Derwent World Patents Index; AN 2006-485292, XP002801277
- DATABASE WPI Week 200630, Derwent World Patents Index; AN 2006-289075, XP002801278
- DATABASE WPI Week 201702, Derwent World Patents Index; AN 2016-534730, XP002801279
- FORTUNIAK WITOLD ET AL: "Route to hydrophilic, hydrophobic and functionalized cross-linked polysiloxane microspheres", POLYMER, ELSEVIER SCIENCE PUBLISHERS B.V, GB, vol. 54, no. 13, 16 April 2013 (2013-04-16), pages 3156 - 3165, XP028558092, ISSN: 0032-3861, DOI: 10.1016/J.POLYMER.2013.04.017

## Beschreibung

Die Anmeldung betrifft ein Verfahren zur Herstellung von Mikropartikeln.

In vielen Bereichen werden Polysiloxane eingesetzt. Es werden immer höhere Ansprüche an diese Materialien gestellt, sodass kommerziell erhältliche Systeme verbessert werden müssen. Eine mögliche Anwendung von Polysiloxanen ist beispielsweise die Verkapselung von optoelektronischen Bauelementen. Die Arbeitsbedingungen von lichtemittierenden Dioden (LEDs) erfordern beispielsweise eine hohe photophysikalische und thermische Stabilität, eine hohe Transparenz, einen hohen Brechungsindex sowie eine gute Verarbeitbarkeit der gehärteten und nicht gehärteten Verkapselungsmaterialien, um eine hohe Effizienz und eine lange Lebensdauer des Bauelements zu gewährleisten.

So werden beispielsweise auf Polysiloxan basierende Verkapselungsmaterialien herangezogen, die auf ZweiKomponenten Elastomer-Systemen basieren und mittels eines Platinkatalysators thermisch härtbar sind. Aus ökologischen und wirtschaftlichen Gründen sollte der Einsatz von nichtrecycelbaren Edelmetallen wie Platin jedoch vermieden werden. Bislang bekannte epoxybasierte Polysiloxane, welche ohne Platin gehärtet werden können, sind jedoch wärme- oder lichtempfindlich, sodass beispielsweise eine Verfärbung aufgrund des Vorhandenseins der Epoxygruppen auftritt. Auch ist oft eine hohe Flexibilität der Materialien erforderlich, welche bislang nicht realisiert werden konnte.

US 2001/025080 A1 betrifft ein Verfahren zur Herstellung von Beschichtungszusammensetzungen auf Wasserbasis, die in der Lage sind, hoch kratz- und abriebfeste matte Beschichtungen zu bilden, in denen vernetzte Silikonteilchen gut dispergiert sind. Das Verfahren beinhaltet die Zugabe einer wässrigen Suspension von vernetzten Silikonteilchen mit einem durchschnittlichen Durchmesser von 0,1-200 µm zu einer Beschichtungszusammensetzung auf Wasserbasis. Das Verfahren ist dadurch gekennzeichnet, dass die Suspension eine wässrige vernetzte Silikonteilchensuspension ist, die durch Bewirken der Vernetzung einer durch Kondensationsreaktion vernetzbaren Silikonzusammensetzung bereitgestellt wird, die (A) ein Organopolysiloxan, das mindestens zwei Silanolgruppen in jedem Molekül enthält, (B) ein Vernetzungsmittel und (C) einen Kondensationsreaktionskatalysator umfasst. Die Vernetzung wird in der emulgierten Zusammensetzung in einer wässrigen Lösung eines anionischen Tensids bewirkt.

Aufgabe mindestens einer Ausführungsform der Erfindung ist es, ein Herstellungsverfahren für ein Polysiloxan-Material mit verbesserten Eigenschaften bereitzustellen.

Diese Aufgaben werden durch das Verfahren nach Anspruch 1 gelöst. Vorteilhafte Ausgestaltungen und Weiterbildungen der Erfindung sind Gegenstand abhängiger Ansprüche und der Beschreibung.

Erfindungsgemäß wird ein Verfahren zur Herstellung von Mikropartikeln bereitgestellt, das die Schritte umfasst:
A) Herstellung einer Lösung eines Tensids in einem polaren Lösungsmittel
B) Zugabe einer schmelzbaren Vorstufe eines Polysiloxan-Glas-Hybrids zur Herstellung einer Mischung aus schmelzbarer Vorstufe und Lösung des Tensids
C) Erhitzen der Mischung über die Schmelztemperatur der schmelzbaren Vorstufe zur Herstellung einer Emulsion, vorzugsweise Mikroemulsion, von Tröpfchen der schmelzbaren Vorstufe, wobei die Größe der Tröpfchen durch Rühren und/oder Ultraschall eingestellt wird
D) Zugabe eines Katalysators zur Initiierung einer Vernetzungsreaktion auf der Oberfläche der Tröpfchen unter Herstellung von Mikropartikeln,
wobei die schmelzbare Vorstufe des Polysiloxan-Glas-+Hybrids ausgewählt ist aus der Gruppe bestehend aus einer der Zusammensetzungen (A), (B) und einer Mischung davon:
(A) ein polymeres Material, das durch Kondensationsreaktionen einer Trialkoxysilanmonomereinheit und/oder einer Dialkoxysilanmonomereinheit hergestellt wurde, wobei die Trialkoxysilanmonomereinheit (3) die Struktur (I) 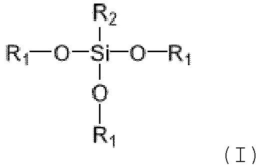 und die Dialkoxysilanmonomereinheit (4) die Struktur (II) 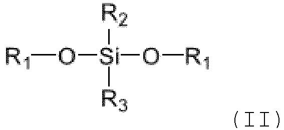 aufweist,
   wobei die Reste R1 und R2 in der Trialkoxysilanmonomereinheit der Struktur (I) unabhängig voneinander aus der Gruppe ausgewählt sind, die aus Cl-C24-alkyl, C3-C24-cycloalkyl, C2-C24-alkine, C7-C24-cycloalkine, C2-C24-alkenyl, Cl-C24-alkoxy, C3-C24-cycloalkoxy, C6-C14-aryl und C6-C14-aryloxy besteht, und wobei die Reste R1, R2 und R3 in der Dialkoxysilanmonomereinheit der Struktur (II) unabhängig voneinander aus der Gruppe ausgewählt sind, die aus Cl-C24-alkyl, C3-C24-cycloaIkyl, C2-C24-alkin, C7-C24-cycloalkin C2-C24-alkenyl, Cl-C24-alkoxy, C3-C24-cycloalkoxy, C6-C14-aryl, C6-C14-aryloxy besteht, wobei vorzugsweise R₁ für die jeweilige Trialkoxysilan- oder Dialkoxysilanmonomereinheit unabhängig voneinander aus Methyl oder Ethyl ausgewählt ist, und wobei vorzugsweise R₂, R₃ für die jeweilige Trialkoxysilan- oder Dialkoxysilanmonomereinheit unabhängig voneinander aus Methyl oder Phenyl ausgewählt sind;
(B) ein Material aufweisend ein dreidimensionales Netzwerk aus teilweise miteinander vernetzten Monomereinheiten und einem alkoxyterminierten Oligo- oder Polysiloxan, wobei die Monomereinheiten zumindest ein Trialkoxysilan und zumindest ein Dialkoxysilan umfassen, wobei das Material vorzugsweise die allgemeine Strukturformel 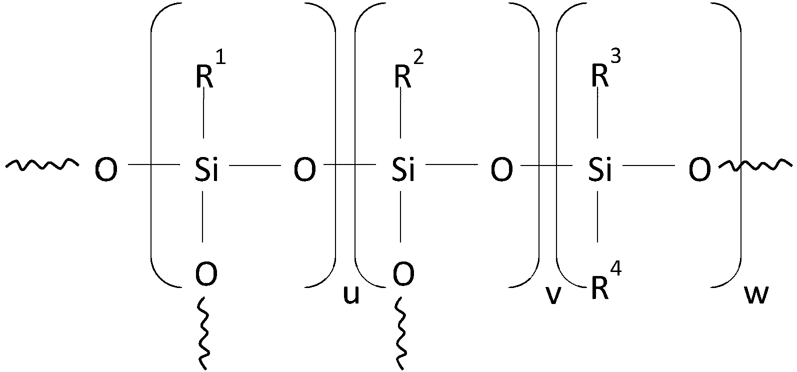 wobei R¹, R², R³ und R⁴ unabhängig voneinander ausgewählt sind aus Aryl, Alkyl, Alkenyl, Allyl, substituiertem Aryl, substituiertem Alkenyl, substituiertem Alkyl und Vinyl, vorzugsweise aus Phenyl und Methyl, wobei u+v+w die Anzahl der eingesetzten Si-Atome ist und u, v und w unabhängig voneinander aus dem Bereich 1 bis 20000 ausgewählt sind, wobei die mit u, v und w indizierten Gruppen statistisch in der allgemeinen Strukturformel verteilt sind.

Unter Mikropartikeln werden Teilchen mit vorzugsweise kugelförmiger Form mit einem Durchmesser von 0,1 bis 100 µm, vorzugsweise von 2 bis 30 µm, weiter bevorzugt 2 bis 20 µm, noch weiter bevorzugt 2 bis 10 µm, insbesondere 3 bis 8 µm, gemessen durch Licht- oder Elektronenmikroskopie verstanden. Entsprechendes gilt für die Form und Größe von Tröpfchen in der erfindungsgemäßen Mikroemulsion.

Dieses Verfahren hat den technischen Vorteil, dass zahlreiche Eigenschaften der Mikropartikel durch das Verfahren eingestellt werden können. So kann die Polarität der Mikropartikel oder deren Oberflächenfunktionalisierung gesteuert werden, wodurch eine große Anzahl kompatibler Matrices zur Verfügung stehen, worin die Mikropartikel eingebettet werden können. So können diese Mikropartikel in unterschiedlichsten Polymermatrices eingesetzt werden um deren Brechungsindex gezielt einzustellen oder um die Fluoreszenzeigenschaften der in den Partikeln integrierbaren Farbstoffe auch in Matrixmaterialien nutzbar zu machen, in denen sich diese Farbstoffe sonst nicht lösen. Zusätzlich bietet die partikuläre Form weitreichende Möglichkeit zur Verwendung in technischen Anwendungen. So können Filme der Partikel auf verschiedensten Substraten hergestellt werden oder durch Dispersion der Partikel Dispersionsfarben oder Druckertinten auf Polysiloxanbasis hergestellte werden. Neben der Verwendung als Verkapselung für Fluoreszenzfarbstoffe ist auch Nutzung als Matrixmaterial für andere organische Substanzen denkbar wie verkapselte Trägermaterialien für Geruchsstoffe, Farben, Arzneimittel oder ähnliches. Durch den Polysiloxan-artigen Aufbau der Materialien, ist hier insbesondere der biologische, pharmazeutische oder kosmetische Sektor als Anwendungsfeld zu sehen.

Vorzugsweise ist die schmelzbare Vorstufe des Polysiloxan-Glas-Hybrids eine bei 20°C und 100 kPa hoch- bis niedrigviskose Flüssigkeit oder ein glasartiger Feststoff. Die schmelzbare Vorstufe fließt bei 100 kPa bei Temperatur T1 und konsolidiert bei Temperatur T2 irreversibel und permanent, wobei T2>T1 und T1, T2 = 70-200°C. Die Konsolidierungsdauer t1 ist abhängig vom gewählten T2 und beträgt vorzugsweise 0,5 bis 95 Stunden. Das Schmelzen bzw. Fließen der schmelzbaren Vorstufen des Polysiloxan-Glas-Hybrids ist kein Schmelzen im thermodynamischen Sinne, sondern ist auf die unvollständige Vernetzung der schmelzbaren Vorstufe zurückzuführen. Die Konsolidierung bei der Temperatur T2 ist auf einen höheren Vernetzungsgrad zurückführbar. Eine partielle Konsolidierung kann ebenfalls durch die Behandlung mit Basen oder Säuren hervorgerufen werden. Die Eigenschaften des nicht konsolidierten und des konsolidierten Materials können über das Verhältnis der eingesetzten Monomere gesteuert werden.

Die schmelzbare Vorstufe des Polysiloxan-Glas-Hybrids ist ein organisch modifiziertes Silica-Gel. Erfindungsgemäß wird die schmelzbare Vorstufe des Polysiloxan-Glas-Hybrids ausgewählt aus der Gruppe bestehend aus einem Material (A), (B) und einer Mischung davon:
(A) ein polymeres Material, das durch Kondensationsreaktionen einer Trialkoxysilanmonomereinheit und/oder einer Dialkoxysilanmonomereinheit hergestellt wurde, wobei die Trialkoxysilanmonomereinheit (3) die Struktur (I) 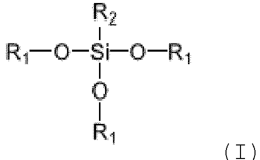 und die Dialkoxysilanmonomereinheit (4) die Struktur (II) 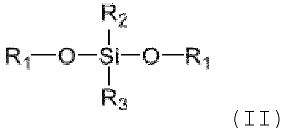 aufweist,
   wobei die Reste R1 und R2 in der Trialkoxysilanmonomereinheit der Struktur (I) unabhängig voneinander aus der Gruppe ausgewählt sind, die aus Cl-C24-alkyl, C3-C24-cycloalkyl, C2-C24-alkine, C7-C24-cycloalkine, C2-C24-alkenyl, Cl-C24-alkoxy, C3-C24-cycloalkoxy, C6-C14-aryl und C6-C14-aryloxy besteht, und wobei die Reste R1, R2 und R3 in der Dialkoxysilanmonomereinheit der Struktur (II) unabhängig voneinander aus der Gruppe ausgewählt sind, die aus Cl-C24-alkyl, C3-C24-cycloaIkyl, C2-C24-alkin, C7-C24-cycloalkin C2-C24-alkenyl, Cl-C24-alkoxy, C3-C24-cycloalkoxy, C6-C14-aryl, C6-C14-aryloxy besteht, wobei vorzugsweise R₁ für die jeweilige Trialkoxysilan- oder Dialkoxysilanmonomereinheit unabhängig voneinander aus Methyl oder Ethyl ausgewählt ist, und wobei vorzugsweise R₂, R₃ für die jeweilige Trialkoxysilan- oder Dialkoxysilanmonomereinheit unabhängig voneinander aus Methyl oder Phenyl ausgewählt sind. Ein Herstellungsverfahren hierfür ist in der Druckschrift EP 3613809 A1 beschrieben. Unter das Material (A) fallen auch Polysilsesquioxane, insbesondere Polyphenylsilsesquioxane, die unter Verwendung von ausschließlich Trialkoxysilanmonomeren erhalten werden können;
(B) ein Material aufweisend ein dreidimensionales Netzwerk aus teilweise miteinander vernetzten Monomereinheiten und einem alkoxyterminierten Oligo- oder Polysiloxan, wobei die Monomereinheiten zumindest ein Trialkoxysilan und zumindest ein Dialkoxysilan umfassen, wobei das Material vorzugsweise die allgemeine Strukturformel 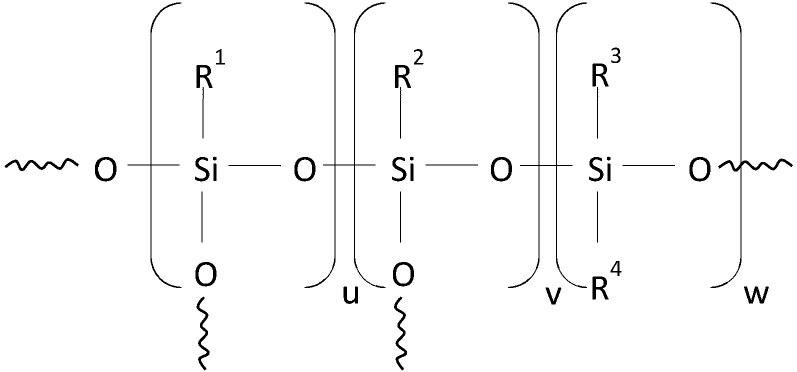 wobei R¹, R², R³ und R⁴ unabhängig voneinander ausgewählt sind aus Aryl, Alkyl, Alkenyl, Allyl, substituiertem Aryl, substituiertem Alkenyl, substituiertem Alkyl und Vinyl, vorzugsweise aus Phenyl und Methyl, wobei u+v+w die Anzahl der eingesetzten Si-Atome ist und u, v und w unabhängig voneinander aus dem Bereich 1 bis 20000 ausgewählt sind, wobei die mit u, v und w indizierten Gruppen statistisch in der allgemeinen Strukturformel verteilt sind. Ein Herstellungsverfahren hierfür ist in der Druckschrift EP 3613793 A1 beschrieben.

Vorzugsweise ist in Schritt A) das polare Lösungsmittel ein polares-protisches Lösungsmittel, vorzugsweise ausgewählt aus der Gruppe bestehend aus C1-C3-Alkohole, vorzugsweise Methanol, Wasser und einer Mischung davon, insbesondere Wasser.

Vorzugsweise ist in Schritt A) das Tensid ein nicht-ionisches Tensid, vorzugsweise ein Alkylphenolethoxylat, oder ein ionisches Tensid, insbesondere Natriumlaurylsulfat (SLS oder SDS) oder Cetyltrimethylammoniumbromid (CTAB).

Vorzugsweise ist in Schritt C) der Katalysator kein Metallkatalysator, vorzugsweise kein Edelmetallkatalysator, insbesondere kein Platinkatalysator und ist vorzugsweise eine Lauge, weiter bevorzugt eine anorganische Base, insbesondere bevorzugt ein Metallhydroxid, beispielsweise Natrium- oder Kaliumhydroxid, oder eine organische Base, beispielsweise eine organische Aminverbindung.

Erfindungsgemäß wird in Schritt C) der Durchmesser der Tröpfchen durch Rühren und/oder Ultraschall, vorzugsweise durch Rühren, mit Hilfe einer Dispergiervorrichtung, eingestellt. Durch Verändern der Rührgeschwindigkeit kann die zahlenbezogene Größe und Größenverteilung der Tröpfchen, gemessen als Durchmesser mit Hilfe einer Licht- oder Elektronenmikroskopie-Aufnahme, und folglich auch der aus den Tröpfchen hergestellten Mikropartikel eingestellt werden. Die Tröpfchen mit vorzugsweise kugelförmiger Form werden vorzugsweise auf einen Durchmesser von 0,1 bis 100 µm, vorzugsweise von 2 bis 30 µm, weiter bevorzugt 2 bis 20 µm, noch weiter bevorzugt 2 bis 10 µm, insbesondere 3 bis 8 µm, gemessen durch Licht- oder Elektronenmikroskopie, eingestellt. Bei Erhöhung der Rührgeschwindigkeit können kleinere Teilchengrößen mit engerer Größenverteilung erhalten werden. Vorzugsweise wird die Emulsion mit einer Rührgeschwindigkeit auf 300 bis 15000 min⁻¹, weiter bevorzugt auf 500 bis 12000 min⁻¹, insbesondere 8000 bis 12000 min⁻¹ für vorzugsweise 1 min bis 5 h, weiter bevorzugt 1 min bis 2 h, insbesondere 2 bis 15 min gerührt.

Vorzugsweise umfasst das Verfahren ferner Schritt E) Isolieren der Mikropartikel, vorzugsweise durch Zentrifugieren, und gegebenenfalls Trocknung unter erhöhter Temperatur und gegebenenfalls vermindertem Druck zur Entfernung des Lösungsmittels umfasst, wobei die Trocknungsbedingungen so gewählt werden, dass auf den Mikropartikeln gebundene Tensidmoleküle gebunden bleiben.

Vorzugsweise erfolgt nach Schritt E) Isolieren der Mikropartikel, eine weitere Erhitzung gegebenenfalls unter vermindertem Druck erfolgt, wobei die Bedingungen so gewählt werden, dass auf den Mikropartikeln gebundene Tensidmoleküle entfernt werden und die Mikropartikel gegebenenfalls konsolidiert werden.

Vorzugsweise ist in die schmelzbare Vorstufe des Polysiloxan-Glas-Hybrids ein Material ausgewählt aus der Gruppe bestehend aus Konversionsmaterial zur Wellenlängenkonversion, vorzugsweise ausgewählt aus der Gruppe bestehend aus organischer Fluoreszenzfarbstoff, vorzugsweise auf Perylendiimid basierend, anorganischer Leuchtstoff und deren Mischung, Geruchsstoff, Farbe, Arzneimittel und einer Mischung davon eingebettet.

Ein Referenzbeispiel betrifft Mikropartikel, die umfassen:
- oberflächenvernetztes Material aus einer schmelzbaren Vorstufe eines Polysiloxan-Glas-Hybrids; und
- an die Oberfläche der oberflächenvernetzten Teilchen gebundene Tensidmoleküle. Die Mikropartikel können nach dem Verfahren nach dem ersten erfindungsgemäßen Aspekt erhalten werden. Sie werden nach der Isolierung nur so weit, gegebenenfalls unter vermindertem Druck, erhitzt, dass Tensidmoleküle auf der Oberfläche der Mikropartikel gebunden bleiben. Die Mikropartikel sind nicht konsolidiert, sind in organischen Lösungsmitteln, wie Toluol, Chloroform, Aceton, Diethylether, vollständig löslich und fließen bei T1.

Vorzugsweise ist die schmelzbare Vorstufe des Polysiloxan-Glas-Hybrids ausgewählt aus der Gruppe bestehend aus einer der vorstehend definierten Materialien (A), (B) und einer Mischung hiervon.

Vorzugsweise ist in die schmelzbare Vorstufe des Polysiloxan-Glas-Hybrids ein Material ausgewählt aus der Gruppe bestehend aus Konversionsmaterial zur Wellenlängenkonversion, vorzugsweise ausgewählt aus der Gruppe bestehend aus organischer Fluoreszenzfarbstoff, vorzugsweise auf Perylendiimid basierend, anorganischer Leuchtstoff und deren Mischung, Geruchsstoff, Farbe, Arzneimittel und einer Mischung davon eingebettet.

Ein weiteres Referenzbeispiel betrifft Mikropartikel umfassend konsolidiertes Material aus einem Polysiloxan-Glas-Hybrid, vorzugsweise basierend auf der schmelzbare Vorstufe des Polysiloxan-Glas-Hybrids, die ausgewählt ist aus der Gruppe bestehend aus einer der vorstehend definierten Materialien (A), (B) und einer Mischung hiervon. Die bei T2 konsolidierten Materialien (sind transparent, thermisch belastbar (>250 °C), hydrophob und in ihrer Härte respektive ihrer Elastizität durch Auswahl der Materialien und gegebenenfalls durch Steuerung des Vernetzungsgrads einstellbar. Zudem sind die Mikropartikel nach dem dritten erfindungsgemäßen Aspekt in organischen Lösungsmitteln, wie Toluol, Chloroform, Aceton, Diethylether, unlöslich.

Vorzugsweise ist in das Polysiloxan-Glas-Hybrids ein Material ausgewählt aus der Gruppe bestehend aus Konversionsmaterial zur Wellenlängenkonversion, vorzugsweise ausgewählt aus der Gruppe bestehend aus organischer Fluoreszenzfarbstoff, vorzugsweise auf Perylendiimid basierend, anorganischer Leuchtstoff und deren Mischung, Geruchsstoff, Farbe, Arzneimittel und einer Mischung davon eingebettet.

Ein weiteres Referenzbeispiel betrifft die Verwendung der im zweiten und/oder dritten erfindungsgemäßen Aspekt definierten Mikropartikel als Film auf verschiedenen Substraten, als Dispersion in Dispersionsfarben oder Druckertinten, als Verkapselung für Fluoreszenzfarbstoffe oder verkapselte Trägermaterialien für Geruchsstoffe, Farben oder Arzneimittel.

Ein weiteres Referenzbeispielbetrifft ein Bauelement aufweisend mindestens ein Bauteil, das Mikropartikel gemäß dem zweiten oder dritten erfindungsgemäßen Aspekt umfasst.

Vorzugsweise ist das Bauelement ein optoelektronisches Bauelement und enthält eine Verkapselung umfassend die Mikropartikel und/oder eine Konversionsschicht umfassend die Mikropartikel.
**Fig. 1** zeigt schematisch die Synthese der Vorstufe eines Polysiloxan-Hybrid-Glases als Material (A) in der Variante eines fluoreszierenden festen Polyphenylsilsesquioxan-Hybridglases.
**Fig. 2** zeigt schematisch die Herstellung von Mikropartikeln aus dem nach dem Schema von Fig. 1 hergestelltem Material.

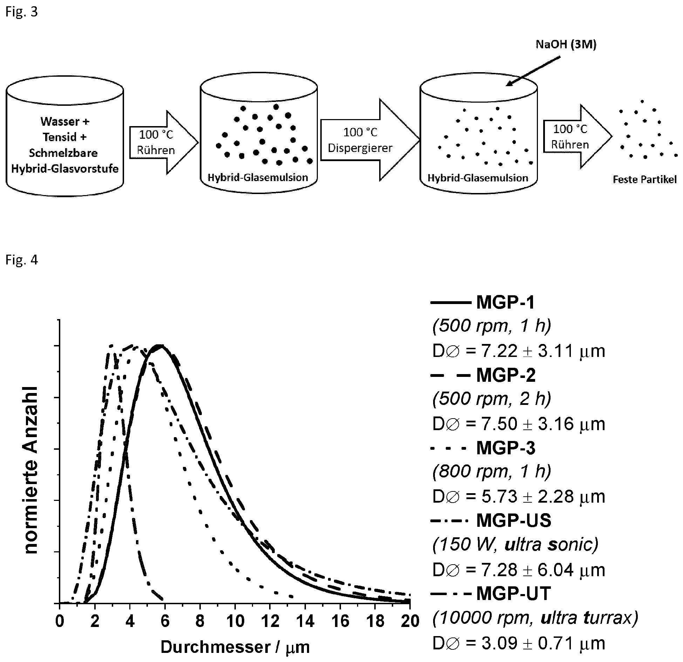

**Fig.** 3 zeigt ein abgewandeltes Verfahren nach Fig. 2, worin zur Steuerung der Teilchengröße und -verteilung eine Dispergiervorrichtung verwendet wird.
**Fig. 4** zeigt den Effekt unterschiedlicher Dispergiervorrichtungen und -verfahren auf die Teilchengröße und -verteilung im Verfahren nach Fig. 3.
**Fig. 5** zeigt die schematische Seitenansicht eines optoelektronischen Bauelements gemäß eines Ausführungsbeispiels.

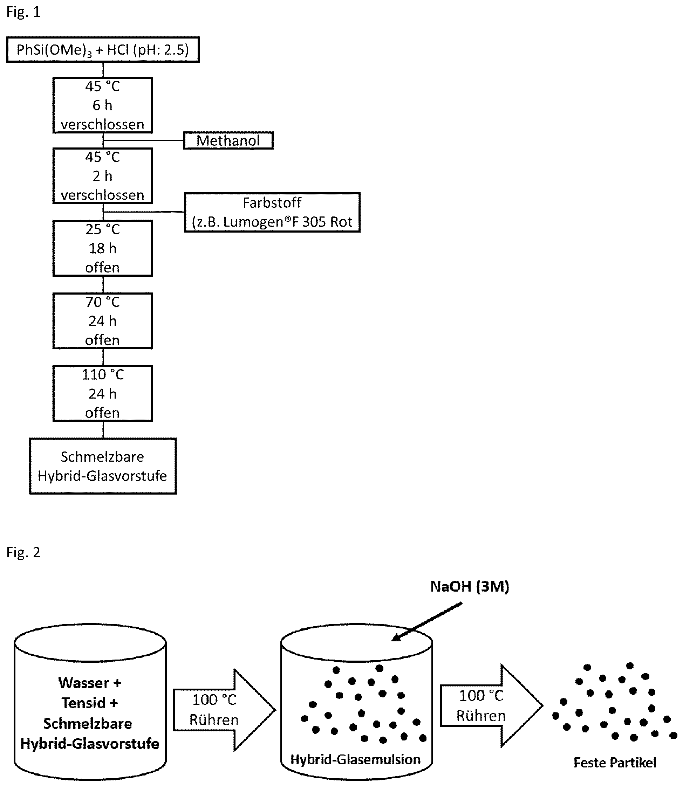

Fig. 1 zeigt schematisch die Synthese der Vorstufe eines Polysiloxan-Hybrid-Glases als Material (A) in der Variante eines fluoreszierenden festen Polyphenylsilsesquioxan-Hybridglases. Dieses wurde ohne Platinkatalysator bei >80 °C geschmolzen und bei >150 °C verfestigt, indem eine säurekatalysierte Kondensationsreaktion von Phenyltrimethoxysilan und anschließende Zugabe von Lumogen^{®}F Red305 vor der Gelierung durchgeführt wurde.

Fig. 2 zeigt schematisch die Herstellung von Mikropartikeln aus dem nach dem Schema von Fig. 1 hergestelltem Material. Das erhaltene Material wurde in eine siedende Lösung von Triton^{™} X-405 überführt, wobei sich eine heiße Emulsion des flüssigen Vorläufers in Wasser bildete. Die Zugabe von Natriumhydroxid zu dieser Emulsion löst eine Kondensation und Verfestigung der Mikropartikel aus. Eine weitere Vernetzung zu einer vollständig kondensierten PhSiO_{1.5}-Struktur und die Entfernung der Triton^{™} X-405-Moleküle von der Oberfläche der Mikropartikel wurde durch eine anschließende Wärmebehandlung der Partikel durchgeführt. Die chemischen Verschiebungen des Polyphenylsilsesquioxan-Hybridglases (MG) ²⁹Si und ¹³C, die durch die Konsistenz der T2- und T3-Einheiten verursacht werden, weisen auf teilweise vernetzte Strukturen hin. Es wurde anschließend bei 200 °C verfestigt, was zu einer noch unvollständigen Kondensation der Struktur führte (Kondensationsgrad: 85 %).

Die FTIR-Spektren der Gase, die durch die Konsolidierung während der TG-Messung freigesetzt werden, zeigen ausschließlich Schwingungen der Kondensationsprodukte Methanol und Wasser. Das konsolidierte Hybridglas (MG Cons.) zeigt keinen weiteren Massenverlust durch die Konsolidierung. Sein T95-Wert wurde bei 488 °C bestimmt. Das Material ist noch teilweise vernetzt, was durch ²⁹Si- und ¹³C-Spektroskopie gezeigt wird.

Die frisch hergestellten und unbehandelten Mikropartikeln (MGP) zeigen ebenfalls teilweise vernetzte Polyphenylsilsesquioxan-Strukturen, die durch chemische Verschiebungen der T2- und T3-Einheiten bei ²⁹Si und ¹³C angezeigt werden (DC: 84 %). Die Partikel sind nicht mehr schmelzbar bzw. fließen nicht, was eine Strukturveränderung und eine weitere Vernetzung durch das Mikropartikel-Syntheseverfahren belegt. Die NMR-Daten der anschließend wärmebehandelten Mikropartikel (MGP-T, 200 °C, 4h) belegen eine vollständige Kondensation zu einem PhSiO_{1,5}-Netzwerk durch die ausschließliche Anwesenheit von T3-Einheiten. Diese Strukturänderung erklärt in erster Linie das Löslichkeitsverhalten dieser Materialien in organischen Lösungsmitteln wie Aceton. Die weniger vernetzten Materialien, die aus T2- und T3-Einheiten bestehen, sind löslich, die vollständig vernetzten und wärmebehandelten MGP-T-Mikropartikeln nicht, und ihre Morphologie bleibt erhalten.

Die Mikropartikel (MGP-T) zeigen eine rote Fluoreszenz mit einem Emissionsmaximum bei 647 nm (15456 cm⁻¹). Die Quantenausbeute von 0,82 ist im Vergleich zu der von MG leicht vermindert, was durch Streu- und Reabsorptionsprozesse verursacht wird, die durch die Änderung der Festphasenmorphologie vom transparenten Hybridglas zum Mikropartikelpulver hervorgerufen werden. Der Farbstoff ist trotz der vollständig vernetzten Struktur der Matrix durch Behandlung mit organischen Lösungsmitteln, wie Aceton, aus den Mikropartikeln auslaugbar. Die Partikel können homogen in härtbare Polysiloxanharze, beispielsweise das in EP 3613793 A1 offenbarte härtbare Polysiloxanharz oder Polyphenylmethylsiloxanharz [Dow Corning OE6630; Dow(1)], eingearbeitet werden und zeigen nach 25 Stunden eine leichte Abnahme der Absorption bei 450 nm durch Bestrahlung mit 450 nm Wellenlänge bei ~0,85 mW/m² und damit eine hohe Photostabilität.

Die mittlere Partikelgrößenverteilung wurde durch fünf Experimente mit unterschiedlichen Rührgeschwindigkeiten oder Energieeintrag optimiert. Die engste Verteilung und die kleinsten Partikel mit 3,09±0,71 µm wurden mit der Dispergiervorrichtung Ultra-Turrax^{™} erhalten.

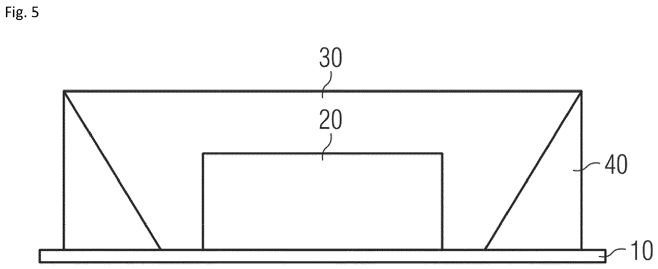

Figur 5 zeigt die schematische Seitenansicht eines optoelektronischen Bauelements gemäß eines Ausführungsbeispiels. Das Bauelement, beispielsweise eine LED, umfasst ein Substrat 10, auf dem eine Halbleiterschichtenfolge 20 angeordnet ist. Die Halbeiterschichtenfolge 20 ist zur Emission von Primärstrahlung, beispielsweise kurzwelliges Licht mit einem Wellenlängenmaximum von etwa 450 nm, eingerichtet.

Im Strahlengang der Primärstrahlung ist eine Konversionsschicht 30 aus gehärteten Polysiloxanharz (z.B. Polyphenylmethylsiloxanharz [Dow Corning OE6630; Dow(1)]) mit darin eingelagerten erfindungsgemäßen konsolidierten Mikropartikeln (MGP-T) umfassend in den Mikropartikeln eingelagertes Lumogen^{®}F Red305 angeordnet. Diese umhüllt die Halbleiterschichtenfolge 20 vollständig, das heißt stoff- und formschlüssig, und ist somit als Verguss in einer Ausnehmung des Gehäuses 40 eingebracht. Damit dient die Konversionsschicht 30 zum einen als Verkapselung der Halbleiterschichtenfolge 20, zum anderen zur Konversion der Primärstrahlung in eine Sekundärstrahlung. Die Konversionsschicht enthält die konsolidierten Mikropartikel, der in einer Matrix, welche aus einer Zusammensetzung gebildet ist, eingelagert ist.

Alternativ kann die Konversionsschicht 30 beabstandet zu der Halbleiterschichtenfolge 20 angeordnet sein (hier nicht gezeigt). In diesem Fall kann zwischen der Halbleiterschichtenfolge 20 und der Konversionsschicht 30 eine Verkapselung, die aus gehärteten Polysiloxanharz (z.B. Polyphenylmethylsiloxanharz [Dow Corning OE6630; Dow(1)) mit darin eingelagerten konsolidierten Mikropartikeln (MGP-T) umfassend in den Mikropartikeln eingelagertes Lumogen^{®}F Red305 gebildet ist, angeordnet sein.

### Beispiel 1: Allgemeine Vorschrift zur Herstellung der schmelzbaren Vorstufe eines Polysiloxan-Glas-Hybrids als Material (A) - Möglichkeit 1

Die Darstellung Materials erfolgt über einen mehrstufigen Prozess. Kein, ein oder mehrere Trialkoxysilanmonomere (TAS) werden mit einem Alkohol (Cl-C12-Alkohol, C2-C12-Diol, C3-C24-CycloalkohoIe. C5-C24-Arylalkohole), mit Wasser und Salzsäure im molaren Verhältnis 1:4:3:0.01 verschlossen gerührt (10-40 °C, 0,5-10 h). Kein, ein oder mehrere Dialkoxysilan- oder Trialkoxysilanmonomere (DAS/TAS) werden im molaren Verhältnis 1:4 mit einem Alkohol (Cl-C12-alkohol, C2-C12-diol, C3-C24-cycloalkohole, C5-C24-arylalkohole) verrührt, dem ersten Gemisch zugetropft, welches weiter gerührt wird (10-40°C, 0,5 - 10 h). Ammoniumhydroxidlösung wird dem Gemisch zugegeben und weiter gerührt (10 - 40 CC, 0,5 - 10 h). Das verschlossene Gefäß wird geöffnet und weiter gerührt bis das Gemisch geliert (10 - 40 °C, 0,5 - 200 h). Das Gemisch wird erwärmt um Alkoholreste zu entfernen (40 - 200 °C, 0,5 - 200 h). Ausgefallenes Ammoniumchlorid wird durch Waschen mit Aceton und Filtration unter vermindertem Druck entfernt (0,5 - 50 ml). Zu dem Gemisch wird eine definierte Menge eines organischen Fluoreszenzfarbstoffs zugegeben und mechanisch eingearbeitet. Das Gemisch wird erwärmt, um Lösungsmittelreste zu entfernen (40 - 110 CC, 0,5 - 200 h). Das Gemisch wird erwärmt, um Wasserreste zu entfernen (110 - 200°C, 0,5-200 h).

### Beispiel 2: Allgemeine Vorschrift zur Herstellung der schmelzbaren Vorstufe eines Polysiloxan-Glas-Hybrids als Material (A) - Möglichkeit 2

Die Darstellung des Materials erfolgt über einen mehrstufigen Prozess. Kein, ein oder mehrere Trialkoxysilanmonomere (TAS) werden mit einem Alkohol (Cl-C12-Alkohol C2-C12-Diol, C3-C24-Cycloalkohole, C5-C24-Arylalkohole) in Salzsäure ((pH - 2,5; n(TAS)/n(H₂0)) = 1/1,5) verschlossen gerührt (10 -40 °C, 0,5 - 10 h). Kein ein oder mehrere Dialkoxysilan- oder Trialkoxysilanmonomere (DAS/TAS) werden im molaren Verhältnis 1:4 mit einem Alkohol (Cl-C12-AIkohol, C2-C12-Diol, C3-C24-Cycloalkohole, C5-C24-Arylalkohole) verrührt, dem ersten Gemisch zugetropft, welches weiter gerührt wird (10-40 °C, 0,5 - 10 h). Das verschlossene Gefäß wird geöffnet und weiter gerührt bis das Gemisch geliert (10 - 40 °C, 0,5 - 200 h). Zu dem Gemisch wird eine definierte Menge eines organischen Fluoreszenzfarbstoffs zugegeben und mechanisch eingearbeitet. Das Gemisch wird erwärmt, um Lösungsmittelreste zu entfernen (40 - 110 °C, 0,5 - 200 h). Das Gemisch wird erwärmt, um Wasserreste zu entfernen (110-200 °C, 0,5 - 200 h).

### Beispiel 3: Allgemeine Vorschrift zur Herstellung der schmelzbaren Vorstufe eines Polysiloxan-Glas-Hybrids als Material (B)

Ein oder mehrere Trialkoxysiianmonomere (TAS) werden mit einem oder mehrere Dialkoxysilanmonomere (DAS) im gewünschten molaren Verhältnis mit Salzsäure (pH: 2,5) im molaren Verhältnis 1:1.5 [Σn(AS):n(HCl] verschlossen gerührt (45°C, 3 h). Dem Gemisch wird eine definierte Menge eines alkoxysilanterminerten Polyalkylsiloxans zugegeben. Es wird weiter verschlossen gerührt (45 °C, 18 h). Das Gemisch wird anschließen in ein Becherglas überführt und solange gerührt bis sich ein Gel bilden, in dem sich stabile Blasen bilden (25 °C). Das Reaktionsgefäß wird dann in einen Ofen transferiert und erhitzt um Wasser und Salzsäure zu entfernen (110 °C, 1 h). Das erhaltene Gel kann nun verarbeitet und erhöhten Temperaturen konsolidiert werden (>160 °C).

### Beispiel 4: Allgemeine Vorschrift zur Darstellung der Mikropartikel

Ein Tensid, zum Beispiel Polyoxyethylene(40)isooctylphenylether (Triton^{™} X-405, 70 wt% in Wasser [ca. 0,356 mmol/g]) wird in Wasser gegeben (14 ml, 0,77 mol) und gerührt (500 min⁻¹, 100 °C). Eine definierte Menge der schmelzbaren Polymervorstufe wird hinzugegeben (z.B. 100 mg). Die sich gebildete Emulsion wird weiter gerührt (0,4 - 2 h, 100 °C). Natriumhydroxidlösung wird zum Reaktionsgemisch zugegeben (1 ml, 3 mol/l). Nach weiterem Rühren (500 min⁻¹, 0,4 h, 100 °C) wird die Emulsion abgekühlt (Eiswasser). Die erhaltenen Mikropartikel werden mittels Zentrifugation isoliert und gewaschen (Wasser, 4 mal) und unter vermindertem Druck getrocknet (80 °C, 500 Pa). Durch eine anschließende Temperaturbehandlung bei >200 °C kann das überschüssige Tensid auf der Partikeloberfläche entfernt werden. Zusätzlich erfolgt eine Nachhärtung des Materials.

### Beispiel 4: Herstellung von Mikropartikeln aus Zusammensetzung (A) in der Variante eines polyphenylsilsesquioxanähnlichem Hybridglases

Die hybriden Glasmikropartikeln wurden in einem zweistufigen Verfahren synthetisiert. Zunächst wurde ein polyphenylsilsesquioxanähnliches Vorläufer-Hybridglas (MG) hergestellt. Während der Synthese wurde ein spezifischer Perylendiimid-Farbstoff zur Einarbeitung in das Material hinzugefügt. Der in dieser Arbeit verwendete Farbstoff war Lumogen^{®}F Rot 305, der physikalisch in das Hybridglas integriert ist.

### Beispiel 4.1: Synthese von Vorläufer-Hybridglas (MG)

Phenyltrimethoxysilan (25 g, 126,07 mmol, [PhSi(OMe)3]) und Salzsäure (pH=2,5, 3,41 g, 189,12 mmol) wurden in einem verschlossenen Fläschchen bei 45 °C 6 h lang gerührt. Absolutes Methanol (1,80 g, 56. 18 mmol) wurde unter weiterem Rühren bei 40 °C für 2 h tropfenweise zugegeben. Die Lösung wurde in ein 100-ml-Becherglas überführt, eine bestimmte Menge eines der Perylendiimid-Farbstoffe wurde hinzugefügt (18 mg, n(LG305) = 16,68 µmol). Die Mischung wurde gerührt, bis die Gelierung abgeschlossen war (25 °C, 18 h). Das Becherglas wurde in einen Kammertrockner überführt und zur Unterbrechung der Reaktionen in zwei Schritten von Wasser, Salzsäure und Methanol befreit. Daher wurde das Material 24 Stunden lang bei 70 °C und danach weitere 24 Stunden bei 110 °C erhitzt. Die roten Materialien wurden auf Raumtemperatur abgekühlt, um feste Hybridglasmaterialien zu erhalten (15,24 g, 1200 ppm/1,09 µmol g⁻¹¬ LG305). Die Hybridgläser erweichen reversibel bei einer Temperatur > 80 °C. Bei Temperaturen > 150 °C verfestigen sich die Materialien irreversibel. Die nicht verfestigten Materialien wurden mit MG (LG305) bezeichnet. Die verfestigten Materialien wurden mit MG Cons (LG305) bezeichnet.

### Beispiel 4.2: Synthese-Hybridglas-Mikropartikeln (MGP)

Polyoxyethylen(40)isooctylphenylether-Lösung (90 mg, Triton^{™} X-405, 70 Gew.-% in Wasser [~0,356 mmol/g]) wurde zu Wasser (14 ml, 0,77 mol) gegeben und bei 100 °C mit 500 U/min gerührt. Die 100 mg des Hybridglasvorläufers wurden der heißen Lösung zugegeben. Die durch das Schmelzen des Vorläufers entstandene Emulsion wurde 0,4 - 2 h bei 100 °C weiter gerührt. Der Emulsion wurde Natriumhydroxidlösung zugegeben (1 ml, 3 mol L⁻¹). Nach weiterem Rühren mit 500 min⁻¹ für 0,4 h bei 100 °C wurde die Emulsion in einem Eiswasserbad abgekühlt. Die erhaltenen Hybridglas-Mikropartikeln wurden isoliert und durch 4-maliges Zentrifugieren mit Wasser gewaschen und in einem Trockenschrank unter reduziertem Druck bei 80 °C und 5 mbar getrocknet. Die erhaltenen Produkte werden als MGP (LG305) bezeichnet. Eine anschließende Wärmebehandlung der Partikel wurde ebenfalls für einen Vergleich mit den unbehandelten durchgeführt. Die wärmebehandelten Partikel wurden als MGP-T (LG305) bezeichnet.

### Beispiel 5: Synthese von Zusammensetzung (B)

In einem Headspace-Vial wurden 5,00 g PhSi(OMe)₃, 1,05 g MeSi(OMe)₃ und 2,46 Me₂Si(OMe)₂ mit 1,45 mL HCl-Lösung (pH 2,5) versetzt und luftdicht verschlossen. Das Reaktionsgemisch wurde dann 3 h bei einer Temperatur von 45 °C bei 500 min⁻¹ gerührt. Danach wurden 0,30 g Polydimethyldimethoxysilan PDMS (PDMSi₁₁(OMe)₂) zugegeben und bei dieser Temperatur weiter gerührt. Anschließend wurde das Vial geöffnet und die Reaktionsmischung für 1 h bei 110 °C im Trockenschrank getrocknet.

### Beispiel 6: Herstellung von Mikropartikeln aus Zusammensetzung (B)

Zu 17,5 mL 6,9 gL⁻¹ Triton X-Lösung wurden 125 mg der Zusammensetzung (B) gegeben und bei einer Rührgeschwindigkeit von 500 min⁻¹ für mindestens 30 min emulgiert. Anschließend wurden 50 µL 3 M NaOH-Lösung hinzugegeben und bei 100 °C für weitere 24 h bei 500 min⁻¹ gerührt. Die Reaktionslösung wurde dann auf Raumtemperatur abgekühlt und mindestens je drei Mal bei 8000 min⁻¹ abzentrifugiert und mit entionisiertem Wasser gewaschen. Daraufhin wurden die Partikel in einem vakuumierten Trockenschrank bei 80 °C für mindestens 24 h getrocknet.

### Ergebnisse und Diskussion

### Polysilsesquioxan-Vorläufer

Fluoreszierende Hybridglas-Mikropartikeln wurden durch Bildung einer oberflächenaktiven Emulsion aus einem farbstoffhaltigen Polyphenylsilsesquioxan-Vorläufer synthetisiert. Der Vorläufer wurde durch eine säurekatalysierte Polykondensationsreaktion von Phenyltrimethoxysilan unter Bildung teilweise vernetzter Strukturen hergestellt (Schema 1).

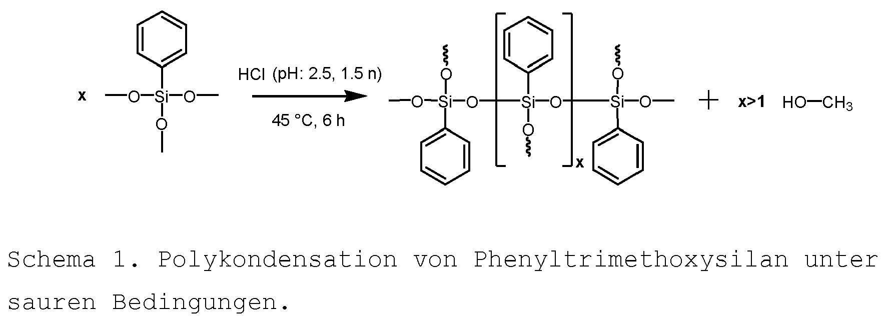

Während eines Gelierungsschrittes können die organischen Fluoreszenzfarbstoffe dem Syntheseverfahren bzw. dem Material zugesetzt werden. Die erhaltenen Proben sind fest, transparent und starr und erweichen reversibel bei Temperaturen > 80 °C.

Der Vorläufer wurde durch FTIR- und NMR-Spektroskopie charakterisiert. Die FTIR-Spektren zeigen die erwarteten Schwingungsbanden eines MG-Polyphenylsilsesquioxan-Netzwerks, die durch breite (Si-O)-Schwingungsbanden bei 1052, 1016, 806 cm⁻¹ und Phenyl-Seitengruppen bei 3050, 3072, 2953, 2910 [(C-H)AR]; 1130 (Si-C)AR; 726 cm⁻¹ [(C-H)] und 694 cm⁻¹ [(Si-C)] angezeigt werden. Zusätzlich werden die Schwingungsbänder bei 3380 (O-H), 2840 [(C-H[O-CH₃])] und 846 [(Si-O-CH₃)] durch die anhängenden und terminalen Methoxygruppen der Polymere sowie durch nicht umgesetzte Monomere, Methanol oder Wasser verursacht. Die ¹H- und ¹³C-NMR-Spektren zeigen die chemischen Verschiebungen der Phenylprotonen [8,07 - 6,84 (m, Phenyl)] und der Alkoxyprotonen [3,96 - 2,97 (m, MeOR [R= H, CH₃])] sowie die entsprechenden chemischen Kohlenstoffverschiebungen bei -134,26 bis -127.93 ppm [Phenyl] und -50.78 ppm [MeOR [R= H, CH₃]. Signale des integrierten Farbstoffs sind aufgrund der geringen Farbstoffkonzentration nicht nachweisbar. Die zweidimensionale ¹H-²⁹Si-HMBC-NMR-Spektroskopie wurde zur Charakterisierung der Vernetzung der Siliciumatome in der Vorstufe verwendet. Zu den chemischen Verschiebungen der trifunktionellen T2-Einheiten von Phenyltrimethoxysilan-Monomeren sind auch T3-Einheiten nachweisbar (Tabelle 1). Die zusätzliche chemische Verschiebung bei -22 ppm kann auf kleine Spuren von Silikonfett zurückgeführt werden.

**Tabelle 1. ²⁹Si und ¹H chemische Verschiebung der Polyphenylsilsesquioxan-Vorstufe, die durch zweidimensionale ¹H-²⁹Si HMBC NMR-Spektroskopie nachgewiesen wurde.**

| ²⁹Si [Ph**Si**(OR)₃₋ₓ] | ¹H [**Ph**Si(OMe)₃ₓ] | ¹H [PhSi(O**Me**)₃₋ₓ] | **Funktionelle Gruppe** |
|---|---|---|---|
| -75...-80 | 8.07...6.84 | - | **T³** |
| -64...-74 | 8.07...6.84 | 3.96...2.97 | **T²** |

Die präparierte Polysilsesquioxan-Vorstufe besteht aus einer teilweise vernetzten Struktur von T2- und T3-Einheiten. Das Material erweicht und schmilzt sogar bei höheren Temperaturen, was auf die Flexibilität der Polymerketten zurückzuführen ist, die aus einem hohen Anteil an linear verbundenen T2-Phenyltrimethoxysilan-Einheiten bestehen. Während des Reaktionsprozesses bildet sich ein vollständig vernetztes Polysilsesquioxan mit der Zusammensetzung PhSiO_{1,5}, aber einige Alkoxygruppen sind noch nicht hydrolysiert, was aus den T2-Einheiten durch die 2D-NMR geschlossen werden kann. Diese Teilvernetzung von Phenyltrialkoxysilanen wurde bereits für Partikel auf Silsesquioxan-Basis berichtet, die durch Sol-Gel-Reaktionen erhalten wurden. Die sperrigen Phenylsubstituenten sollten aus sterischen Gründen die Bildung dichter T3-Netzwerke verhindern. Der Kreuzpeak der chemischen Verschiebung der T2-Einheiten und der Methoxy-Protonen im ²⁹Si-NMR-Verfahren bestätigt ebenfalls das Vorhandensein von nicht umgesetzten Methoxygruppen an den Siliciumatomen. Die Anzahl der verbleibenden Methoxygruppen wurde durch die Änderung der Intensität der chemischen ¹H-Methoxyverschiebung vor und nach der Synthese berechnet. 10 % der ursprünglichen Menge an Methoxygruppen sind noch im Polymer vorhanden. Die Anzahl der T2-Einheiten kann nicht aus den NMR-Daten berechnet werden, da hydrolysierte und nicht hydrolysierte Einheiten im ¹H-Spektrum nicht getrennt werden können und aufgrund des ¹H-²⁹Si HMBC-Experiments eine quantitative Auswertung der ²⁹Si-NMR-Peakflächen nicht sinnvoll ist.

### Vorläufer-Konsolidierung

Das Vorprodukt ist in organischen Lösungsmitteln löslich und kann durch eine zusätzliche Wärmebehandlung bei Temperaturen >200°C thermisch verfestigt werden. Die Verfestigung wird durch die Kondensation noch vorhandener Methoxy- und Hydroxygruppen verursacht, die in dem teilvernetzten Vorprodukt verbleiben. Nach der Verfestigung ist das Material nicht mehr schmelzbar, aber noch in organischen Lösungsmitteln löslich. Die thermische Analyse des Vorläufers zeigt die Unterschiede im Verhalten des Materials vor und nach der thermischen Verfestigung. Die Wärmebehandlung des Vorläufers führt zu einer irreversiblen Verfestigung des Materials, die durch eine weitere Kondensation von T2-Einheiten verursacht wird. Die TG-Messung von MG zeigt einen Massenverlust von 3% ab 216 °C, verursacht durch diese Kondensationsreaktion. Die FTIR-Spektren der bei diesen Temperaturen freigesetzten Gase zeigen ausschließlich Schwingungen des Kondensationsprodukts Methanol und Wasser bei 3082 [(O-H)], 2980, 1467 [(C-H [O-CH₃])], 1033 cm⁻¹[(C-O)]. Nach diesem Ereignis nimmt die Signalintensität der Methanolschwingungen ab, und das Material ist irreversibel verfestigt.

Es wurde kein zusätzlicher Massenverlust durch weitere Kondensationsreaktionen oder durch gebildete Nebenprodukte, wie Cyclosiloxane, beobachtet, was in der Literatur für vergleichbare Kondensationsreaktionen von Materialien, die Tri- und Dialkoxysilanmonomere enthalten, beschrieben wurde. Nach diesem Verfestigungsschritt beginnt der Abbau von MG Cons. bei 488 °C, definiert durch den T95-Wert, bei dem 95 % der Ausgangsmasse verbleiben. Die ATR-FTIR-Spektren des verfestigten Materials zeigen keine relevanten Veränderungen im Vergleich zum unverfestigten Material, lediglich die Wasserschwingungen bei 3380 cm⁻¹ [(O-H)] sind verschwunden. Im ²⁹Si-NMR-Spektrum der verfestigten Probe sind noch T3- und T2-Einheiten mit -80 und -72 ppm sowie Kohlenstoff aus verbleibenden Methoxygruppen in der und ¹³C CP-MAS-NMR bei -50,38 ppm nachweisbar. Dies lässt den Schluss zu, dass das Material durch die Wärmebehandlung irreversibel verfestigt, aber danach nicht vollständig vernetzt ist. Eine semiquantitative Analyse der Peakflächen der chemischen Verschiebungen in der ²⁹Si CP-MAS-NMR durch Anpassung einer Lorentz-Funktion führt zu einem Gesamtprozentsatz von T3-Einheiten von 55 % und einem Kondensationsgrad (DC) von 85 %. Diese Bildung teilvernetzter Strukturen aus T2- und T3-Einheiten durch thermische Verfestigung wurde in der Literatur bereits für dialkoxysilanhaltige Hybridgläser auf Polysiloxanbasis diskutiert.

### Hybride Glas-Mikropartikel

Für die anschließende Bildung von Mikropartikel wurde die nicht verfestigte Polysilsesquioxan-Vorstufe einer siedenden Lösung eines nichtionischen Tensids in Wasser zugesetzt (Triton^{™} X-405). Das Material wird weich, und es bildet sich eine heiße Emulsion.

Die hydrophoben Eigenschaften des integrierten Farbstoffs auf Perylenbasis lassen ihn in der Vorläuferphase gelöst bleiben, ohne dass er in die Lösung ausgelaugt wird. Durch die Zugabe von Natriumhydroxid zur Lösung beginnen die Oberflächenmoleküle der Materialien durch basenkatalysierte Hydrolyse- und Kondensationsreaktionen von Hydroxygruppen mit den Siliciumatomen zu vernetzen (Schema 2). 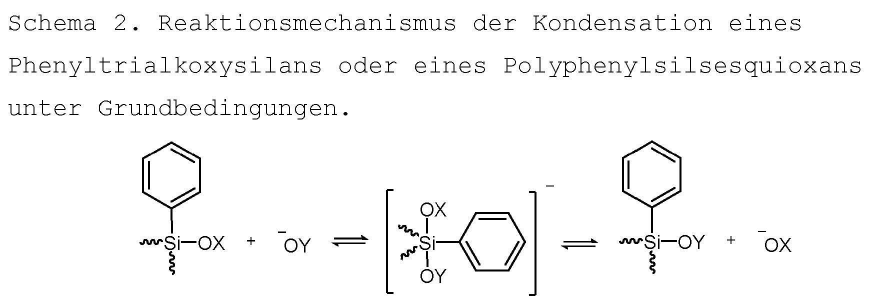
Hydrolyse-Reaktion: X = Me, Y = H
Kondensationsreaktion: X = Me oder H, Y = PhSi(OR)₃

Infolgedessen beginnen die Tropfen, sich chemisch zu konsolidieren, wobei ihre sphärische Morphologie erhalten bleibt. Nach Abkühlung der Lösung auf Raumtemperatur können die Mikropartikel durch Zentrifugation isoliert werden. Nachdem die Partikel isoliert und bei 80°C unter reduziertem Druck getrocknet wurden, veränderte sich die Farbe des Materials aufgrund von Lichtstreueffekten von rot zu rosa. Die erhaltenen Mikropartikel sind in Wasser oder Ethanol unlöslich und werden noch von dem bei der Synthese verwendeten Tensid beschichtet. Das Tensid bewirkt, dass die Kugel hydrophil ist. Eine weitere Wärmebehandlung bei 200 °C führt zur Entfernung des Tensids bzw. einer hydrophoberen Kugeloberfläche. Gleichzeitig wird die Mikrokugelstruktur weiter vernetzt, was das Material auch in organischen Lösungsmitteln unlöslich macht.

Eine Variation der mittleren Kugelgröße wurde durch Änderung der Rührmethode und der Rührgeschwindigkeit zur Synthese unterschiedlich großer Kugeln realisiert. Die Eigenschaften des Hybridglas-Vorläufers (MG) und der Hybridglas-Mikropartikel (MGP) wurden mit 1D- und 2D-Kernresonanzspektroskopie (1H, 29Si und 1H-29Si HMBC 2D-NMR, MAS-NMR), Infrarotspektroskopie (ATR-FTIR) sowie thermogravimetrischen Methoden (TG, TG-FTIR) untersucht.

Die Größe und Morphologie der Mikropartikel wurde lichtmikroskopisch untersucht, und die optischen Eigenschaften des integrierten Farbstoffs wurden mittels Fluoreszenzspektroskopie und Quantenausbeute untersucht. Bestimmung.

Eine anschließende Wärmebehandlung bei 200 °C für 4 h wurde ebenfalls durchgeführt, um die Eigenschaften wie Struktur, Löslichkeit und Oberflächenbeschichtung mit unzerstörten Mikropartikel zu vergleichen. Die unbehandelten Mikropartikel werden mit MGP und die zusätzlich wärmebehandelten mit MGP-T bezeichnet. Zur weiteren Charakterisierung der Struktur des Materials wurden ²⁹Si und ¹³C CP-MAS-NMR, sowie FTIR-Spektroskopie und thermische Analysemethoden durchgeführt. Die ATR-FTIR-Spektren von MG und MGP-T zeigen keine signifikanten Unterschiede im Vergleich zum Vorläufer oder zum konsolidierten Vorläufer der Mikropartikel. Außerdem konnten keine Schwingungsbänder, die durch eine Oberflächenbeschichtung von MG mit Triton^{™} X-405 verursacht wurden, nachgewiesen werden. Auch die Morphologie der MGP-T-Partikel wird durch die Wärmebehandlung nicht beeinflusst.

In den ¹³C-MAS-NMR-Spektren von MGP konnte das Tensid durch die chemischen Verschiebungen, die durch die Triton^{™} X-405 Struktur erzeugt werden, nachgewiesen werden. Die chemischen Verschiebungen bei und 133, 129, 127 ppm (C-H [Phenoxy/Phenyl]) werden durch die aromatischen Protonen der Phenyl- und Phenoxygruppen der Polyphenylsilsesquioxan-Struktur und das Tensid erzeugt. Weitere Verschiebungen des Tensids werden bei 70 (O-CH₂), 37 (C-CH₂) und 31 ppm (C-CH₃) nachgewiesen. Die zusätzliche chemische Verschiebung bei 49 ppm wird durch die nicht umgesetzten Methoxygruppen der T2-Einheiten des Polysilsesquioxans verursacht. Das Vorhandensein dieser Verschiebung deutet auch darauf hin, dass die chemische Konsolidierung ebenfalls zu teilweise vernetzten Strukturen führt. Nach der Wärmebehandlung nahmen die Signalintensität des Tensids und die chemischen Verschiebungen der Methoxygruppen ab oder verschwanden sogar.

Dieser Befund wird durch die ²⁹Si CP-MAS-NMR-Daten bestätigt. Im NMR-Spektrum der chemisch konsolidierten MGP-Mikropartikel können T2- und T3-Einheiten bei -71 und -79 ppm nachgewiesen werden. Der berechnete Prozentsatz der T3-Einheiten beträgt 53 %, und der DC-Wert liegt bei 84 %, was etwas weniger ist als für die thermisch konsolidierte Vorstufe berechnet. Bei anderen thermisch unbehandelten Partikeln auf Polyphenylsilsesquioxan-Basis schwankt der DC zwischen 40 - 90 %. Der Grund für die teilweise vernetzte Struktur wurde oben diskutiert.

Im Spektrum der thermisch verfestigten MGP-T-Partikel kann nur die chemische Verschiebung T3 bei -79 ppm sicher zugeordnet werden, was auf eine vollständig vernetzte PhSiO_{1.5}-Struktur hinweist. Es zeigt sich, dass sich der thermische Verfestigungsprozess der Mikropartikel von dem des Vorläufers unterscheidet, in dem danach noch T2-Einheiten vorhanden sind. Es zeigt auch einen direkten Einfluss der Vorbehandlung der Kugeln mit Natriumhydroxid auf die weitere Strukturbildung. Die anschließende thermische Behandlung führt zu einem Vernetzungsgrad von 100 %. Dadurch können zwei verschiedene Prozesse untersucht werden.

Nachdem die Basis die chemische Konsolidierung der flüssigen Vorläufertropfen initialisiert hat, ist ihre Struktur teilweise vernetzt und ihre Oberfläche mit einer Schicht des Tensids Triton^{™} X-45 beschichtet. Dies führt zu einer hydrophilen, in Wasser sehr gut dispergierbaren Oberfläche. Nach der Wärmebehandlung wird das Tensid von der Oberfläche entfernt, und die T2-Methoxy- und Hydroxygruppen kondensieren zu einer vollständig vernetzten Polyphenylsilsesquioxan-Struktur mit nur T3-Einheiten. Die Partikel können nicht mehr homogen in Wasser suspendiert werden, was darauf hinweist, dass die Oberfläche nun hydrophil ist.

Die Löslichkeit der unbehandelten MGP-Materialien ist gleich der des Vorläufermaterials. Die unbehandelten MGP-Mikropartikel mit teilweise vernetzter Struktur sind in organischen Lösungsmitteln löslich, die zusätzlich wärmebehandelten und vollständig vernetzten MGP-T-Partikel sind unlöslich, und ihre sphärische Morphologie bleibt erhalten. Diese Vielfalt in der Löslichkeit wird auch durch den unterschiedlichen Vernetzungsgrad der Mikropartikel verursacht, wie oben diskutiert. Die MGP-T-Mikropartikel bestehen aus voll vernetzter PhSiO_{1,5}-Struktur, während die MGP-Mikropartikel teilweise vernetzt sind. Der integrierte Farbstoff wird aus beiden Materialstrukturen ausgelaugt, wie in Aceton für MGP und MGP-T gezeigt. Bei Anwendungen, bei denen die Partikel in lösungsmittelfreien Medien oder in polaren Lösungsmitteln wie Wasser oder Ethanol eingesetzt werden, wird der Farbstoff nicht aus der Partikelstruktur ausgelaugt. Aufgrund des Auslaugverhaltens wurden REM-Aufnahmen von den frisch hergestellten und von den temperaturbehandelten Partikeln aufgenommen, um die Morphologie der Oberflächenstruktur im Detail zu sehen. Die Partikelstruktur ist glatt, und es gibt keine Veränderung der Morphologie durch die Wärmebehandlung. Es gibt einige Löcher in einigen der Partikel, aber es ist keine poröse Struktur auf diesen Bildern erkennbar. Ein Grund für die Auslaugung des Farbstoffs können Bereiche sein, in denen die Struktur weniger engmaschig ist. Das T3-Netzwerk hat noch etwas Platz, in dem der Farbstoff durch die Partikelstruktur diffundieren kann. Um die Oberfläche noch besser charakterisieren zu können, wird ein TEM-Bild mit höherer Auflösung benötigt.

### Thermische Analyse von Mikropartikel

Die ¹³C MAS-NMR-Daten zeigten das Vorhandensein einer Triton^{™} X-405 Schicht auf der Oberfläche der Mikropartikel nach der chemischen Konsolidierung und Isolierung, die durch den thermisch initiierten Nachkondensationsprozess dieser Kugeln bei 200 °C für 4 h entfernt wird. Durch eine thermische Analyse der nicht manipulierten Mikropartikel MGP wurde dieser Prozess verifiziert. Es wurde ein Massenverlust von 5% festgestellt, beginnend bei 213 °C. Dieser Massenverlust konnte der Entfernung von Triton^{™} X-405 mittels FTIR-Spektroskopie zugeordnet werden. Die bei 2863 [(O-H)], 2358 [(C-H)] und 1745, 1164, 669 cm⁻¹[(C-O)] nachgewiesenen Schwingungsbänder konnten Triton^{™} X-405 zugeordnet werden. Dies wurde durch eine isotherme TG-FTIR-Messung der MGP-Mikropartikel und der verwendeten Lösung Triton^{™} X-405 bei 220 °C verifiziert. Die IR-Spektren des von den Partikeln freigesetzten Gases und der Lösung Triton^{™} X-405 sind identisch.

Die thermisch verfestigten MGP-T-Mikropartikel zeigen keinen Massenverlust mehr, der durch weitere Kondensationsreaktionen verursacht wird. Nach diesem Verfestigungsschritt beginnt die Zersetzung der Materialien bei 438 °C, definiert durch den T95-Wert. Er ist im Vergleich zu MG Cons. aufgrund des pulverförmigen Zustands leicht vermindert. Infolgedessen kann die Triton^{™} X-405-Schicht auf der Mikrokugeloberfläche durch eine Wärmebehandlung bei 200 °C leicht entfernt werden, was durch den in den TG-FTIR-Spektren gezeigten Massenverlust von 5% verifiziert wird. Gleichzeitig wird die Partikelstruktur, wie zuvor beschrieben, vollständig vernetzt.

### Rühreinfluss auf die Partikelgrößenverteilung

Während der Synthese der Mikropartikel wird eine Emulsion gebildet. Durch einfaches Rühren der Emulsion mit verschiedenen Rührgeschwindigkeiten unter Verwendung eines Standard-Laborrührers kann eine enge Größenverteilung erreicht werden. Zur Optimierung der Partikelgröße und - verteilung wurden fünf zusätzliche Experimente durch Änderung der Rührgeschwindigkeit, der Geschwindigkeit oder der Methode durchgeführt (Tabelle 2). Die Rührgeschwindigkeit mit einem Standardstab wurde von 500 (MGP-1) auf 800 min⁻¹ (MGP-3) und die Rührzeit von 1 h (MGP-1) auf 2 h (MGP-2) bei 500 min⁻¹ geändert.

**Tabelle 2. Probenname und Unterschiede in der Rührgeschwindigkeit und -zeit, die für die Synthese der Hybridglas-Mikropartikel verwendet wurden.**

| **Probe** | **Rührrate** /min⁻¹ | | **Rührdauer / min** | **Mittlere Teilchengröße / µm** |
|---|---|---|---|---|
| ***MGP-1*** | 500 | | 1 h | 7.22 ± 3.11 |
| ***MGP-2*** | 500 | | 2 h | 7.50 ± 3.16 |
| ***MGP-3*** | 800 | | 1 h | 5.73 ± 2.28 |
| ***MGP-US*** | | 1. 500 | 10 min | 7.28 ± 6.04 |
| | | 2. Nur Ultraschall (150 W) | 2 min | |
| ***MGP-UT*** | | 1. 500 | 10 min | 3.09 ± 0.71 |
| | | 2. 10000 (Dispergiervorrichtung) | 2 min | |

Unter Verwendung eines Ultra-Schallfingers (150 W [MGP-US]) oder einer Ultra-Turrax^{™} Dispergiervorrichtung (10000 min⁻¹ [MGP-UT]) nach 10-minütigem Rühren bei 500 min⁻¹ wurde der Einfluss von Hochenergieprozessen auf die Partikelmorphologie untersucht. Alle Experimente wurden mit der heißen Emulsion des Vorläufermaterials durchgeführt, bevor das Natriumhydroxid hinzugefügt wurde, um die chemische Verfestigung zu starten bzw. eine Suspension zu bilden.

Die Partikelgröße und -verteilung wurde durch Messung und Zählung von Partikeln aus Mikroskopiebildern bestimmt. Alle gemessenen Partikel sind unabhängig vom Verfahren kleiner als **20** µm. Durch einfaches Rühren der Emulsion bei 500 min⁻¹ für 1 oder 2 h (MGP-1/2) ändert sich die Partikelgrößenverteilung nicht signifikant. Durch Erhöhung der Rührgeschwindigkeit auf 800 min⁻¹ wird die Verteilung enger und die Partikelgröße nimmt ab (MGP-3). Die zusätzliche Ultraschallbehandlung führt zu einer breiteren Verteilung und größeren Partikelgrößen (MGP-US). Die engste Verteilung und die kleinsten Partikel wurden mit dem Dispenser Ultra-Turrax^{™} (MGP-UT) erreicht. Bei Verwendung eines Standard-Rührstabes führt eine Erhöhung der Rührgeschwindigkeit zu einer Verringerung der Partikelgröße und -verteilung. Eine Erhöhung der Rührzeit bei gleicher Geschwindigkeit führt zu keiner signifikanten Änderung der Partikelgröße. Bei anderen auf Sol-Gel basierenden Partikelherstellungsverfahren führt eine ultraschallvermittelte Synthese ebenfalls zu einer Verringerung der Partikelgrößen. Die Verwendung eines Ultraschallfingers während unserer Synthese führt zu einer erhöhten Partikelgrößenverteilung. Ein Grund dafür ist das Fehlen von mechanischem Rühren während der Sonifikation und die hohe Viskosität der flüssigen Hybridglasemulsionstropfen. Die endgültige Tröpfchengrößenverteilung ist im Allgemeinen gleich der Verteilung direkt nach einem Tröpfchenaufschluss durch mechanische Energie und hängt von der Phasenviskosität der Tröpfchen ab. Die hohe Viskosität behindert den Aufschluss der Tröpfchen durch die Einführung von Ultraschallenergie. Durch die Verwendung der Dispergiervorrichtung werden kleine Partikel mit einer engen Verteilung gebildet. Der Grund dafür ist die hohe Rotationsgeschwindigkeit des Rotors, die extrem starke Scherund Schubkräfte erzeugt, die die Tröpfchen zerreißen. Dadurch wird mechanische Energie benötigt, um durch die Zertrümmerung kleinere Partikel zu bilden. Die Energie, die durch Ultraschallwellen in das System eingebracht wird, fördert nicht die Zerkleinerung der Tröpfchen.

### Fluoreszenz und Quanteneffizienz

Der Perylenfarbstoff Lumogen^{®}F Rot 305 wurde im Gelierungsschritt seiner Synthese in das Polyphenylsilsesquioxan-Hybridglas integriert (siehe experimenteller Abschnitt). Der Farbstoff löste sich aufgrund seiner sehr guten Löslichkeit in phenylhaltigen Materialien sofort im Polymer. Während der Herstellung der Emulsion bleibt der Farbstoff in der Polymerphase gelöst. Die isolierten und wärmebehandelten Mikropartikel (MGP-T) zeigen eine rote Fluoreszenz. Bei 500 nm (20000 cm⁻¹) Anregungswellenlänge sind zwei Emissionsbanden bei 622 (16077 cm⁻¹) und 647 nm (15456 cm⁻¹) nachweisbar. Bei 650 nm (15384 cm⁻¹) Emissionswellenlänge sind drei Anregungsbanden bei 578 nm (17301 cm⁻¹), 530 nm (18867 cm⁻¹) und 440 nm (22727 cm⁻¹) nachweisbar. Im Vergleich zu den Fluoreszenzspektren von Lumogen^{®}F Red 305 in Toluol tritt eine kleine bathochrome Verschiebung der Emission auf.187 Die Stokes-Verschiebung in Toluol wurde mit 29 nm (840 cm⁻¹) in den Mikropartikel bestimmt, sie beträgt 44 nm (1224 cm⁻¹). Die absolute Quantenausbeute der Mikropartikel betrug 0,87 (λex=570 nm) und 0,82 (λex=450 nm). Im Vorläufermaterial (MG LG305) und in anderen härtbaren Polyphenylmethylsiloxanen ist die Quantenausbeute von Lumogen^{®}F Rot 305 mit 1,00 etwas höher (λex=450 nm). Eine Partikelmorphologie und -größe bewirkt eine Abnahme des Quants in der Kieselsäure, und über Polymerpartikel wurde bereits berichtet. Der Grund für die verschobene Emission und die verringerte Quantenausbeute sind Reabsorptionsprozesse in der Probe, die durch die relativ hohe Farbstoffkonzentration (1 µmol g⁻¹) verursacht werden, und spektrale Verzerrungen, die durch Streu- und Reabsorptionsprozesse verursacht werden, die durch die Änderung der Festphasenmorphologie von transparentem Hybridglas zu einem Mikropartikelpulver hervorgerufen werden. Unvergleichbar zu Polyphenylmethylsiloxanen mit integriertem Lumogen^{®}F Red 305 ist hier keine Änderung der Quantenausbeute selbst bei einer hohen Konzentration von 3000 ppm nachweisbar.

### Integration von Partikeln in ein Polyphenylmethylsiloxan-Harz

Für den Einsatz in einer organischen Hybrid-LED-Anwendung müssen die hybriden Glasmikropartikel homogen mit einem Silikonharz verkapselt werden. Danach müssen die Partikel und der integrierte Farbstoff den Betriebsbedingungen des LED-Gerätes standhalten, insbesondere der hohen Strahlung bei 450 nm Wellenlänge. Eine erste Abschätzung der Langzeit-Photostabilität wurde durch ein Langzeit-Bestrahlungsexperiment ermittelt. Daher wurden die temperaturbehandelten MGP-3T in Polyphenylmethylsiloxanharz [Dow Corning OE6630; Dow(1)] integriert, das in einen Aluminiumrahmen gegossen und bei 150°C für 4 h thermisch gehärtet wurde. Die fertige Probe [Dow(1)MGP-3T] zeigt eine homogene Verteilung der Partikel im Inneren der Probe und eine starke Fluoreszenz unter Lichteinwirkung bei einer Wellenlänge von 450 nm. Außerdem laugte der Farbstoff verfahrensbedingt nicht aus den Partikeln aus.

Die Probe und eine Probe des verfestigten Vorläufer-Hybridglases wurden mit einer 450 nm-Lichtquelle für >35 h bei ~0,85 W/cm² bestrahlt. Um die photophysikalische Stabilität des eingearbeiteten Farbstoffs zu untersuchen, wurde die Absorption der Probe während des Bestrahlungsvorgangs periodisch gemessen. Die relative Absorption zum Ausgangswert bei 450 nm wurde bestimmt. Die Abnahme der Absorption des verfestigten Hybridglases und der Hybridglas-Mikropartikel im Inneren des gehärteten Polysiloxanharzes ist innerhalb des Experiments nahezu gleich. Sie nimmt nach ~990 h (~39 d) um 10 % ab. Das Hybridglas und die Hybridglaspartikel zeigen eine signifikante Erhöhung der Photostabilität der eingebauten Farbstoffe im Vergleich zu den kommerziell erhältlichen Polysiloxanproben, bei denen LG305 direkt in das Material eingearbeitet wurde. Dort nahm die Absorption der Farbstoffe nach 25 Stunden in Dow Corning OE6630 um 10% ab.

Der Hauptunterschied der Materialien ist der Vernetzungsgrad, der auch die Sauerstoffdurchlässigkeit und damit die Photostabilität der Farbstoffe beeinflussen kann. Die Polysiloxanharze sind durch Hydrosilylierung vernetzt und noch elastisch. Das Hybridglas ist durch Polykondensation von Trialkoxysilanen hochgradig vernetzt, wodurch es hart und steif und wahrscheinlich weniger durchlässig für Sauerstoff ist. Dies wurde auch durch Gasbarriere-Experimente mit anderen Hybridglas-Zusammensetzungen angezeigt. Es wurde auch gezeigt, dass die Änderung der Morphologie von einem Hybridglas zu Mikropartikeln die Photostabilitätseigenschaften des Farbstoffs nicht beeinflusst. Daher sind die farbstoffhaltigen Mikropartikel für den Einsatz in organischen Hybrid-LED-Anwendungen geeignet.

Als Ergebnis konnte die Integration eines hydrophoben Perylenfarbstoffs in Polyphenylsilsesquioxan-Mikropartikel realisiert werden, was zu einem hoch fluoreszierenden Mikrokugelpulver führte. Unter üblichen Bedingungen wie in optoelektronischen Anwendungen oder in der medizinischen Sensorik oder Bildgebung, wo normalerweise Wasser als Lösungsmittel oder Polymere als Wirtsmaterial verwendet werden, bleibt der Farbstoff in der Partikelstruktur. Wie zuvor beschrieben, kann der Farbstoff durch Behandlung mit organischen Lösungsmitteln aus den Mikropartikeln ausgelaugt werden, während die Integrität der Mikropartikel erhalten bleibt. Die Partikel können auch in härtbare Polysiloxane und damit in organische Hybrid-LED-Anwendungen eingekapselt werden.

## Patentansprüche

1. Verfahren zur Herstellung von Mikropartikeln umfassend die Schritte:
A) Herstellung einer Lösung eines Tensids in einem polaren Lösungsmittel
B) Zugabe einer schmelzbaren Vorstufe eines Polysiloxan-Glas-Hybrids zur Herstellung einer Mischung aus schmelzbarer Vorstufe und Lösung des Tensids
C) Erhitzen der Mischung über die Schmelztemperatur der schmelzbaren Vorstufe zur Herstellung einer Mikroemulsion von Tröpfchen der schmelzbaren Vorstufe, wobei die Größe der Tröpfchen durch Rühren und/oder Ultraschall eingestellt wird
D) Zugabe eines Katalysators zur Initiierung einer Vernetzungsreaktion auf der Oberfläche der Tröpfchen unter Herstellung von Mikropartikeln,
wobei die schmelzbare Vorstufe des Polysiloxan-Glas-Hybrids ausgewählt ist aus der Gruppe bestehend aus einer der Zusammensetzungen (A), (B) und einer Mischung davon:
(A) ein polymeres Material, das durch Kondensationsreaktionen einer Trialkoxysilanmonomereinheit und/oder einer Dialkoxysilanmonomereinheit hergestellt wurde, wobei die Trialkoxysilanmonomereinheit (3) die Struktur (I) 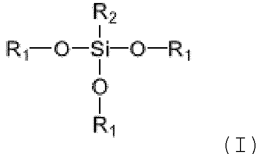 und die Dialkoxysilanmonomereinheit (4) die Struktur (II) 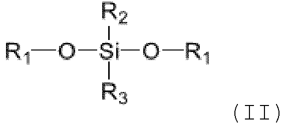 aufweist, wobei die Reste R1 und R2 in der Trialkoxysilanmonomereinheit der Struktur (I) unabhängig voneinander aus der Gruppe ausgewählt sind, die aus Cl-C24-alkyl, C3-C24-cycloalkyl, C2-C24-alkine, C7-C24-cycloalkine, C2-C24-alkenyl, Cl-C24-alkoxy, C3-C24-cycloalkoxy, C6-C14-aryl und C6-C14-aryloxy besteht, und wobei die Reste R1, R2 und R3 in der Dialkoxysilanmonomereinheit der Struktur (II) unabhängig voneinander aus der Gruppe ausgewählt sind, die aus Cl-C24-alkyl, C3-C24-cycloaIkyl, C2-C24-alkin, C7-C24-cycloalkin C2-C24-alkenyl, Cl-C24-alkoxy, C3-C24-cycloalkoxy, C6-C14-aryl, C6-C14-aryloxy besteht, wobei vorzugsweise R₁ für die jeweilige Trialkoxysilan- oder Dialkoxysilanmonomereinheit unabhängig voneinander aus Methyl oder Ethyl ausgewählt ist, und wobei vorzugsweise R₂, R₃ für die jeweilige Trialkoxysilan- oder Dialkoxysilanmonomereinheit unabhängig voneinander aus Methyl oder Phenyl ausgewählt sind;
(B) ein Material aufweisend ein dreidimensionales Netzwerk aus teilweise miteinander vernetzten Monomereinheiten und einem alkoxyterminierten Oligo- oder Polysiloxan, wobei die Monomereinheiten zumindest ein Trialkoxysilan und zumindest ein Dialkoxysilan umfassen, wobei das Material vorzugsweise die allgemeine Strukturformel 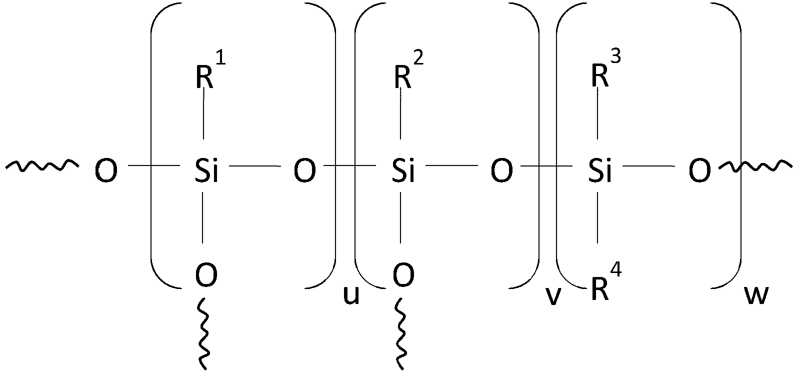 wobei R¹, R², R³ und R⁴ unabhängig voneinander ausgewählt sind aus Aryl, Alkyl, Alkenyl, Allyl, substituiertem Aryl, substituiertem Alkenyl, substituiertem Alkyl und Vinyl, vorzugsweise aus Phenyl und Methyl, wobei u+v+w die Anzahl der eingesetzten Si-Atome ist und u, v und w unabhängig voneinander aus dem Bereich 1 bis 20000 ausgewählt sind, wobei die mit u, v und w indizierten Gruppen statistisch in der allgemeinen Strukturformel verteilt sind.

2. Verfahren zur Herstellung von Mikropartikeln nach Anspruch 1, wobei die schmelzbare Vorstufe des Polysiloxan-Glas-Hybrids eine bei 20°C und 100 kPa hoch- bis niedrigviskose Flüssigkeit oder glasartiger Feststoff ist, wobei die schmelzbare Vorstufe vorzugsweise bei 100 kPa bei Temperatur T1 fließt und bei Temperatur T2 irreversibel und permanent konsolidiert, wobei T2>T1 und T1, T2 = 70-200°C.

3. Verfahren zur Herstellung von Mikropartikeln nach einem der vorhergehenden Ansprüche, wobei in Schritt A) das polare Lösungsmittel ein polares-protisches Lösungsmittel, vorzugsweise Wasser, ist.

4. Verfahren zur Herstellung von Mikropartikeln nach einem der vorhergehenden Ansprüche, wobei in Schritt A) das Tensid ein nicht-ionisches Tensid, vorzugsweise ein Alkyphenolethoxylat, oder ein ionisches Tensid ist.

5. Verfahren zur Herstellung von Mikropartikeln nach einem der vorhergehenden Ansprüche, wobei in Schritt D) der Katalysator kein Metallkatalysator, vorzugsweise kein Edelmetallkatalysator, insbesondere kein Platinkatalysator ist und vorzugsweise eine Lauge, weiter bevorzugt ein Metallhydroxid, insbesondere Natrium- oder Kaliumhydroxid, ist.

6. Verfahren zur Herstellung von Mikropartikeln nach einem der vorhergehenden Ansprüche, wobei in Schritt C) die Größe der Tröpfchen mit Hilfe einer Dispergiervorrichtung, vorzugsweise mit einer Rührgeschwindigkeit auf 300 bis 15000 min⁻¹, weiter bevorzugt auf 500 bis 12000 min⁻¹, insbesondere 8000 bis 12000 min⁻¹ für vorzugsweise 1 min bis 5 h, weiter bevorzugt 1 min bis 2 h, insbesondere 2 bis 15 min eingestellt wird.

7. Verfahren zur Herstellung von Mikropartikeln nach einem der vorhergehenden Ansprüche, wobei das Verfahren ferner Schritt E) Isolieren der Mikropartikel, vorzugsweise durch Zentrifugieren, und gegebenenfalls Trocknung unter erhöhter Temperatur und gegebenenfalls vermindertem Druck zur Entfernung des Lösungsmittels umfasst, wobei die Trocknungsbedingungen so gewählt werden, dass auf den Mikropartikeln gebundene Tensidmoleküle gebunden bleiben.

8. Verfahren zur Herstellung von Mikropartikeln nach Anspruch 7, wobei nach Schritt E) Isolieren der Mikropartikel, eine weitere Erhitzung gegebenenfalls unter vermindertem Druck erfolgt, wobei die Bedingungen so gewählt werden, dass auf den Mikropartikeln gebundene Tensidmoleküle entfernt werden und die Mikropartikel gegebenenfalls konsolidiert werden.

9. Verfahren zur Herstellung von Mikropartikeln nach einem der vorhergehenden Ansprüche, wobei in die schmelzbare Vorstufe des Polysiloxan-Glas-Hybrids ein Material ausgewählt aus der Gruppe bestehend aus Konversionsmaterial zur Wellenlängenkonversion, vorzugsweise ausgewählt aus der Gruppe bestehend aus organischer Fluoreszenzfarbstoff, vorzugsweise auf Perylendiimid basierend, anorganischer Leuchtstoff und deren Mischung, Geruchsstoff, Farbe, Arzneimittel und einer Mischung davon eingebettet ist.

## Claims

1. Method for the preparation of microparticles comprising the steps of:
A) Preparation of a solution of a surfactant in a polar solvent
B) Addition of a meltable precursor of a polysiloxane-glass hybrid for the preparation of a mixture of meltable precursor and solution of the surfactant
C) Heating the mixture above the melting temperature of the meltable precursor for the preparation of a microemulsion of droplets of the meltable precursor, the size of the droplets being adjusted by stirring and/or ultrasound
D) Addition of a catalyst to initiate a cross-linking reaction on the surface of the droplets for the preparation of microparticles,
wherein the meltable precursor of the polysiloxane-glass hybrid is selected from the group consisting of one of the compositions (A), (B) and a mixture thereof:
(A) a polymeric material prepared by condensation reactions of a trialkoxysilane monomer unit and/or a dialkoxysilane monomer unit, wherein the trialkoxysilane monomer unit (3) has the structure (I) 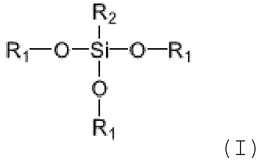 and the dialkoxysilane monomer unit (4) has the structure (II) 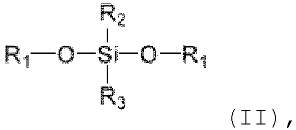 wherein the residues R1 and R2 in the trialkoxysilane monomer unit of structure (I) are independently selected from the group consisting of Cl-C24-alkyl, C3-C24-cycloalkyl, C2-C24-alkyne, C7-C24-cycloalkyne, C2-C24-alkenyl, Cl-C24-alkoxy, C3-C24-cycloalkoxy, C6-C14-aryl and C6-C14-aryloxy, and wherein the residues R1, R2 and R3 in the dialkoxysilane monomer unit of structure (II) are independently selected from the group consisting of Cl-C24-alkyl, C3-C24-cycloalkyl, C2-C24-alkyne, C7-C24-cycloalkyne, C2-C24-alkenyl, Cl-C24-alkoxy, C3-C24-cycloalkoxy, C6-C14-aryl, C6-C14-aryloxy, wherein preferably R₁ for the respective trialkoxysilane or dialkoxysilane monomer unit is independently selected from methyl or ethyl, and wherein preferably R₂, R₃ for the respective trialkoxysilane or dialkoxysilane monomer unit are independently selected from methyl or phenyl;
(B) a material comprising a three-dimensional network of partially cross-linked monomer units and an alkoxy-terminated oligo- or polysiloxane, said monomer units comprising at least one trialkoxysilane and at least one dialkoxysilane, said material preferably having the general structural formula 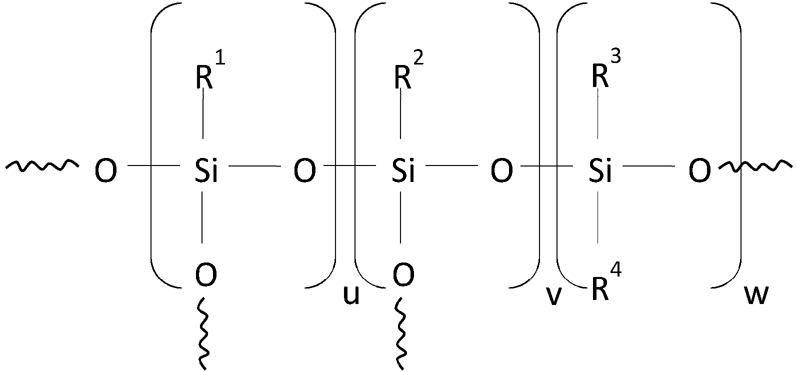 wherein R¹, R², R³ and R⁴ are independently selected from aryl, alkyl, alkenyl, allyl, substituted aryl, substituted alkenyl, substituted alkyl and vinyl, preferably from phenyl and methyl, wherein u+v+w is the number of Si atoms employed and u, v and w are independently selected from the range 1 to 20000, wherein the groups indexed by u, v and w are statistically distributed in the general structural formula.

2. Method for the preparation of microparticles according to claim 1, wherein the meltable precursor of the polysiloxane-glass hybrid is a liquid or glassy solid of high to low viscosity at 20°C and 100 kPa, the meltable precursor preferably flowing at 100 kPa at temperature T1 and irreversibly and permanently consolidating at temperature T2, wherein T2>T1 and T1, T2 = 70-200°C.

3. Method for the preparation of microparticles according to any of the preceding claims, wherein in step A) the polar solvent is a polar protic solvent, preferably water.

4. Method for the preparation of microparticles according to any of the preceding claims, wherein in step A) the surfactant is a non-ionic surfactant, preferably an alkyphenol ethoxylate, or an ionic surfactant.

5. Method for the preparation of microparticles according to any of the preceding claims, wherein in step D) the catalyst is not a metal catalyst, preferably not a noble metal catalyst, in particular not a platinum catalyst, and is preferably an alkali, more preferably a metal hydroxide, in particular sodium or potassium hydroxide.

6. Method for the preparation of microparticles according to any of the preceding claims, wherein in step C) the size of the droplets is adjusted with the aid of a dispersing device, preferably with a stirring speed of 300 to 15000 min⁻¹, more preferably 500 to 12000 min⁻¹, in particular 8000 to 12000 min⁻¹ for preferably 1 min to 5 h, more preferably 1 min to 2 h, in particular 2 to 15 min.

7. Method for the preparation of microparticles according to any of the preceding claims, wherein the process further comprises step E) isolating the microparticles, preferably by centrifugation, and optionally drying under elevated temperature and optionally reduced pressure to remove the solvent, wherein the drying conditions are chosen such that surfactant molecules bound on the microparticles remain bound.

8. Method for the preparation of microparticles according to claim 7, wherein after step E) isolation of the microparticles, further heating is carried out, optionally under reduced pressure, the conditions being selected such that surfactant molecules bound on the microparticles are removed and the microparticles are optionally consolidated.

9. Method for the preparation of microparticles according to any of the preceding claims, wherein a material selected from the group consisting of conversion material for wavelength conversion, preferably selected from the group consisting of organic fluorescent dye, preferably based on perylenediimide, inorganic phosphor and a mixture thereof, odorant, color, drug and a mixture thereof is embedded in the meltable precursor of the polysiloxane-glass hybrid.

## Revendications

1. Procédé de préparation de microparticules comprenant les étapes consistant à:
A) Préparation d'une solution d'un agent de surface dans un solvant polaire
B) Ajout d'un précurseur fusible d'un hybride polysiloxane-verre pour préparer un mélange de précurseur fusible et de solution de l'agent de surface
C) Chauffage du mélange au-dessus de la température de fusion du précurseur fusible pour produire une microémulsion de gouttelettes du précurseur fusible, la taille des gouttelettes étant ajustée par agitation et/ou ultrasons
D) Ajout d'un catalyseur pour initier une réaction de réticulation à la surface des gouttelettes, produisant ainsi des microparticules,
dans lequel le précurseur fusible de l'hybride polysiloxane-verre est choisi dans le groupe constitué par l'une des compositions (A), (B) et un mélange de celles-ci:
(A) un matériau polymère préparé par des réactions de condensation d'une unité monomère de trialcoxysilane et/ou d'une unité monomère de dialcoxysilane, dans lequel l'unité monomère de trialcoxysilane (3) a la structure (I) 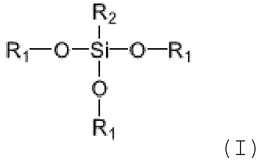 et l'unité monomère de dialcoxysilane (4) a la structure (II) 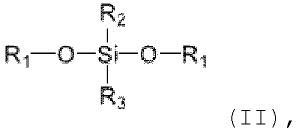 dans laquelle les radicaux R1 et R2 dans l'unité monomère de trialcoxysilane de structure (I) sont choisis indépendamment dans le groupe constitué par un alkyle en Cl-C24, cycloalkyle en C3-C24, alcynes en C2-C24, cycloalcynes en C7-C24, alcényle en C2-C24, Cl-C24-alcoxy, C3-C24-cycloalcoxy, C6-C14-aryle et C6-C14-aryloxy, et où les radicaux R1, R2 et R3 dans l'unité monomère de dialcoxysilane de structure (II) sont choisis indépendamment dans le groupe constitué par alkyle en C1-C24, cycloalkyle en C3-C24, alcyne en C2-C24, cycloalcyne en C7-C24, alcényle en C2-C24, alcoxy en Cl-C24, cycloalcoxy en C3-C24, aryle en C6-C14, aryloxy en C6-C14, de préférence R₁ étant choisi indépendamment parmi les groupes méthyle ou éthyle pour le unité monomère trialcoxysilane ou dialcoxysilane respectif, et dans lequel, de préférence, R₂, R₃ pour le unité monomère de trialcoxysilane ou de dialcoxysilane respectif sont choisis indépendamment parmi les groupes méthyle ou phényle ;
(B) un matériau comprenant un réseau tridimensionnel de unités monomères partiellement réticulés et un oligo- ou polysiloxane à terminaison alcoxy, les dits unités monomères comprenant au moins un trialcoxysilane et au moins un dialcoxysilane, ledit matériau ayant de préférence la formule structurale générale 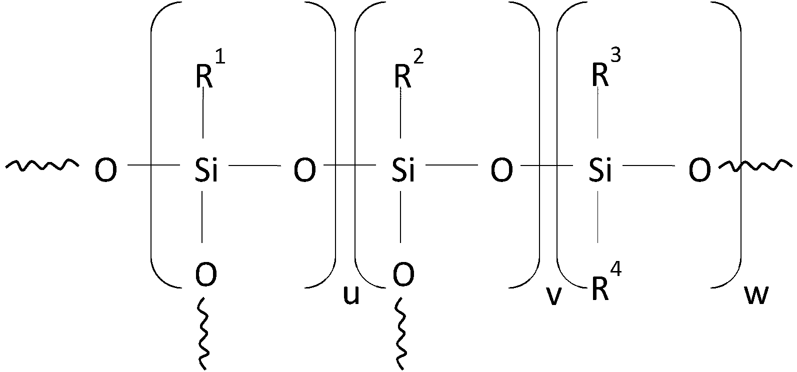 dans laquelle R¹, R², R³ et R⁴ sont choisis indépendamment parmi les groupes aryle, alkyle, alcényle, allyle, aryle substitué, alcényle substitué, alkyle substitué et vinyle, de préférence parmi les groupes phényle et méthyle, u+v+w étant le nombre d'atomes de Si utilisés et u, v et w étant choisis indépendamment dans la plage de 1 à 20000, les groupes indiqués par u, v et w étant distribués de manière statistique dans la formule structurale générale.

2. Procédé de préparation de microparticules selon la revendication 1, dans lequel le précurseur fusible de l'hybride polysiloxane-verre est un liquide ou un solide vitreux de haute à basse viscosité à 20°C et 100 kPa, le précurseur fusible s'écoulant de préférence à 100 kPa à la température T1 et se consolidant de manière irréversible et permanente à la température T2, où T2>T1 et T1, T2 = 70-200°C.

3. Procédé de préparation de microparticules selon l'une quelconque des revendications précédentes, dans lequel, dans l'étape A), le solvant polaire est un solvant polaireprotique, de préférence de l'eau.

4. Procédé de préparation de microparticules selon l'une quelconque des revendications précédentes, dans lequel, dans l'étape A), le agent de surface est un agent de surface non ionique, de préférence un éthoxylate d'alkylphénol, ou un agent de surface ionique.

5. Procédé de préparation de microparticules selon l'une quelconque des revendications précédentes, dans lequel, à l'étape D), le catalyseur n'est pas un catalyseur métallique, de préférence un catalyseur à base de métal noble, en particulier un catalyseur à base de platine, et est de préférence une base, de préférence encore un hydroxyde métallique, en particulier l'hydroxyde de sodium ou de potassium.

6. Procédé de préparation de microparticules selon l'une quelconque des revendications précédentes, dans lequel, à l'étape C), la taille des gouttelettes est ajustée à l'aide d'un dispositif de dispersion, de préférence avec une vitesse d'agitation comprise entre 300 et 15000 min⁻¹, plus préférentiellement entre 500 et 12000 min⁻¹, en particulier entre 8000 et 12000 min⁻¹, pendant de préférence 1 minute à 5 heures, plus préférentiellement 1 minute à 2 heures, en particulier 2 à 15 minutes.

7. Procédé de préparation de microparticules selon l'une quelconque des revendications précédentes, ledit procédé comprenant en outre l'étape E) d'isolement des microparticules, de préférence par centrifugation, et éventuellement de séchage à température élevée et éventuellement sous pression réduite pour éliminer le solvant, les conditions de séchage étant choisies de manière à ce que les molécules de d'agent de surface liées sur les microparticules restent liées.

8. Procédé de préparation de microparticules selon la revendication 7, dans lequel, après l'étape E), on isole les microparticules, on procède à un chauffage supplémentaire, éventuellement sous pression réduite, les conditions étant choisies de telle sorte que les molécules d'agent de surface fixées sur les microparticules soient éliminées et que les microparticules soient éventuellement consolidées.

9. Procédé de préparation de microparticules selon l'une quelconque des revendications précédentes, dans lequel est incorporé dans le précurseur fusible de l'hybride polysiloxane-verre un matériau choisi dans le groupe constitué par un matériau de conversion pour la conversion de longueur d'onde, de préférence choisi dans le groupe constitué par un colorant fluorescent organique, de préférence à base de pérylènediimide, une substance fluorescente inorganique et son mélange, une substance odorante, une peinture, un médicament et un mélange de ceuxci.
